# EUROPEAN PATENT SPECIFICATION

(11) **EP 2 094 696 B1**
(45) Date of publication and mention of the grant of the patent: **13.10.2010**
(21) Application number: 07819889.2
(22) Date of filing: 21.11.2007
(51) Int. Cl.: C07D 471/04, A61K 31/522, A61P 29/00

(54) **5-SULFANYLMETHYL-[1,2,4] TRIAZOL[1, 5-A] PYRIMIDIN-7-OL DERIVATIVES AS CXCR2 ANTAGONISTS**
5-SULFANYLMETHYL[1,2,4]TRIAZOL[1,5-A]PYRIMIDIN-7-OLDERIVATE ALS CXCR2-ANTAGONISTEN
DÉRIVÉS DE 5-SULFANYLMÉTHYL-[1,2,4] TRIAZOL[1, 5-A] PYRIMIDIN-7-OL UTILISÉS COMME ANTAGONISTES DE CXCR2

(30) Priority: 23.11.2006 EP 06124679
(43) Date of publication of application: 02.09.2009
(73) Proprietor: Novartis AG, 4002 Basel (CH)
(72) Inventor: PRESS, Neil John, Horsham, West Sussex RH12 5AB (GB); PORTER, David, Horsham, West Sussex RH12 5AB (GB); SPANKA, Carsten, 79541 Lörrach (DE)
(74) Representative: Vögeli-Lange, Regina
(86) International application number: PCT/EP2007/010098
(87) International publication number: WO 2008/061741

(56) References cited:
- EP-A- 1 676 834
- WO-A-2005/070906
- WO-A-2005/113534

## Description

The present invention relates to triazolopyrimidines, e.g. compounds of formula (I), and uses thereof.

In one aspect the present invention provides a compound of formula 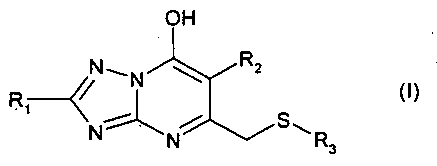 wherein
R₁ and R₂ independently are hydrogen, (C₁₋₈)alkyl, (C₃₋₈)cycloalkyl, (C₁₋₈)alkylthio, (C₆₋₁₈)aryl, (C₁₋₈)alkyl(C₆₋₁₈)aryl, (C₆₋₁₈)aryl(C₁₋₈)alkyl, heterocyclyl having 5 to 6 ring members and 1 to 4 heteroatoms selected from N, O, S,
R₃ is (C₆₋₁₈)aryl or heterocyclyl having 5 to 6 ring members and 1 to 4 heteroatoms selected from N, O, S, unsubstituted or 1- or morefold substituted by (C₁₋₆)alkyl, halo(C₁₋₆)alkyl, halogen, halo(C₁₋₆)alkoxy, cyano, unsubstituted or substituted phenyl, heterocyclyl having 5 to 6 ring members and 1 to 4 heteroatoms selected from N, O, S,
or a pharmaceutically acceptable salt or solvate thereof.

In another aspect the present invention provides a compound of formula (I) wherein
R₁ and R₂ independently are hydrogen, (C₁₋₄)alkyl, (C₃₋₆)cycloalkyl, (C₁₋₄)alkylthio, phenyl, phenyl(C₁₋₄)alkyl, heterocyclyl having 5 to 6 ring members and 1 to 4 heteroatoms selected from N, O, S,
R₃ is phenyl, naphthyl or heterocyclyl having 6 ring members and 1 to 2 heteroatoms selected from N, O, S, unsubstituted or 1- or 3-fold substituted by (C₁₋₄)alkyl, halogen, halo(C₁₋₄)alkyl, halo(C₁₋₄)alkoxy, cyano, phenyl, heterocyclyl having 5 to 6 ring members and 1 to 2 heteroatoms selected from N, O, S.

In another aspect the present invention provides a compound of formula (I) wherein
R₁ is hydrogen, methyl, ethyl, i-propyl, cyclopropyl, cyclohexyl, methylthio, phenyl, benzyl, phenethyl,
R₂ is hydrogen,
R₃ is unsubstituted naphthyl or phenyl, phenyl 1- to 3-fold substituted by methyl, chloro, fluoro, trifluoromethyl, trifluoromethoxy, phenyl, phenoxy, 4-methylphenyl, unsubstituted naphthyl, pyridinyl substituted by trifluoromethyl or 2-5-dimethylfuran-3-yl.

In a compound of formula (I) R₁ preferably is hydrogen, methyl, ethyl, i-propyl, cyclopropyl, cyclohexyl, methylthio, phenyl, benzyl, phenethyl.
In a compound of formula (I) R₂ preferably is hydrogen.
In a compound of formula (I) R₃ preferably is is unsubstituted naphthyl or phenyl, phenyl 1-or 3-fold substituted by methyl, chloro, fluoro, trifluoromethyl, trifluoromethoxy, phenyl, phenoxy, 4-methylphenyl, unsubstituted naphthyl, pyridinyl substituted by trifluoromethyl or 2-5-dimethylfuran-3-yl.

In a compound of formula I each single defined substitutent may be a preferred substituent, e.g. independently of each other substitutent defined.

If not otherwise defined herein
- alkyl includes linear or branched (C₁₋₈)alkyl, such as (C₁₋₆)alkyl or (C₁₋₄)alkyl, e.g. (C₁₋₂)alkyl, including unsubstituted or substituted alkyl, e.g. alkyl substituted by groups which are conventional in organic chemistry, e.g. halogen, OH, NH₂ or halo(C₁₋₆)alkyl, e.g. methyl, ethyl, propyl, i-propyl, butyl;
- cycloalkyl includes (C₃₋₈)cycloalkyl, such as (C₃₋₆)cycloalkyl, e.g. cyclopropyl, cyclohexyl;
- halogen includes fluoro, chloro, bromo, iodo, e.g. fluoro, chloro, bromo, preferably fluoro or chloro;
- alkoxy includes (C₁₋₄)alkoxy, such as (C₁₋₂)alkoxy, e.g. methoxy;
- alkylthio includes (C₁₋₈)alkylthio, such as (C₁₋₄)alkylthio, e.g. methylthio;
- aryl includes (C₆₋₁₈)aryl, e.g. phenyl, naphthyl, and (C₆₋₁₈)aryl(C₁₋₈)alkyl, e.g. benzyl, phenethyl, unsubstituted or 1- or morefold, e.g. 1- to 3-fold, substituted by groups which are conventional in organic chemistry, e.g. halogen, such a s chloro or fluoro, (C₁₋₆)alkyl, e.g. methyl, or halo(C₁₋₆)alkyl, e.g. trifluoromethyl; optionally annelated with heterocyclyl having 5 or 6 ring members and 1 to 4 heteroatoms selected from N, O, S;
- heterocyclyl includes heterocyclyl having 5 or 6 ring members and 1 to 4 heteroatoms selected from N, O, S, preferably N, O, such as alicyclic and aromatic heterocyclyl, e.g. heterocyclyl having 6 ring members and 1 to 2 heteroatoms selected from N, O, S, such as pyridinyl, furanyl, unsubstituted or substituted, e.g. substituted by methyl.

In another aspect the present invention provides a compound selected from the group consisting of
- 2-Benzyl-5-(2,3-difluorophenylsulfanylmethyl)-[1,2,4]triazolo[1,5-a]pyrimidin-7-ol
- 2-Methyl-5-(4-chlorophenylsulfanylmethyl)-[1,2,4]triazolo[1,5-a]pyrimidin-7-ol
- 2-Methyl-5-(4-methylphenylsulfanylmethyl)-[1,2,4]triazolo[1,5-a]pyrimidin-7-ol
- 5-(2,3-difluorophenylsulfanylmethyl)-[1,2,4]triazolo[1,5-a]pyrimidin-7-ol
- 2-Methyl-5-(2,3-difluorophenylsulfanylmethyl)-[1,2,4]triazolo[1,5-a]pyrimidin-7-ol
- 2-Ethyl-5-(2,3-difluorophenylsulfanylmethyl)-[1,2,4]triazolo[1,5-a]pyrimidin-7-ol
- 2-Isopropyl-5-(2,3-difluorophenylsulfanylmethyl)-[1,2,4]triazolo[1,5-a]pyrimidin-7-ol
- 2-Cyclopropyl-5-(2,3-difluorophenylsulfanylmethyl)-[1,2,4]triazolo[1,5-a]pyrimidin-7-ol
- 2-Methylthio-5-(2,3-difluorophenylsulfanylmethyl)-[1,2,4]triazolo[1,5-a]pyrimidin-7-ol
- 2-Phenethyl-5-(2,3-difluorophenylsulfanylmethyl)-[1,2,4]triazolo[1,5-a]pyrimidin-7-ol
- 2-Phenyl-5-(2,3-difluorophenylsulfanylmethyl)-[1,2,4]triazolo[1,5-a]pyrimidin-7-ol
- 2-Cyclohexyl-5-(2,3-difluorophenylsulfanylmethyl)-[1,2,4]triazolo[1,5-a]pyrimidin-7-ol
- 2-Benzyl-5-phenylsulfanylmethyl-[1,2,4]triazolo[1,5-a]pyrimidin-7-ol
- 2-Benzyl-5-(3,4-difluorophenylsulfanylmethyl)-[1,2,4]triazolo[1,5-a]pyrimidin-7-ol
- 2-Benzyl-5-(5-trifluoromethyl-pyridin-2-yl-sulfanylmethyl)-[1,2,4]triazolo[1,5-a]pyrimidin-7-ol
- 2-Benzyl-5-(2,3-dichlorophenylsulfanylmethyl)-[1,2,4]triazolo[1,5-a]pyrimidin-7-ol
- 2-Benzyl-5-(3-trifluoromethyl-phenylsulfanylmethyl)-[1,2,4]triazolo[1,5-a]pyrimidin-7-ol
- 2-Benzyl-5-(naphthalen-2-yl-sulfanylmethyl)-[1,2,4]triazolo[1,5-a]pyrimidin-7-ol
- 2-Benzyl-5-(3,4-dichlorophenylsulfanylmethyl)-[1,2,4]triazolo[1,5-a]pyrimidin-7-ol
- 2-Benzyl-5-(3,5-dichloro-phenylsulfanylmethyl)-[1,2,4]triazolo[1,5-a]pyrimidin-7-ol
- 2-Benzyl-5-(2,4-dichloro-phenylsulfanylmethyl)-[1,2,4]triazolo[1,5-a]pyrimidin-7-ol
- 2-Benzyl-5-(3-trifluoromethoxy-phenylsulfanylmethyl)-[1,2,4]triazolo[1,5-a]pyrimidin-7-ol
- 2-Benzyl-5-(4-chloro-2,5-dimethyl-phenylsulfanylmethyl)-[1,2,4]triazolo[1,5-a]pyrimidin-7-ol
- 2-Benzyl-5-(4-phenoxy-phenylsulfanylmethyl)-[1,2,4]triazolo[1,5-a]pyrimidin-7-ol
- 2-Benzyl-5-(2,5-difluoro-phenylsulfanylmethyl)-[1,2,4]triazolo[1,5-a]pyrimidin-7-ol
- 2-Benzyl-5-(4'-methyl-biphenyl-3-ylsulfanylmethyl)-[1,2,4]triazolo[1,5-a]pyrimidin-7-ol
- 2-Benzyl-5-(3-fluoro-4-methyl-phenylsulfanylmethyl)-[1,2,4]triazolo[1,5-a]pyrimidin-7-ol and
- 2-Benzyl-5-(2,5-dimethyl-furan-3-ylsulfanylmethyl)-[1,2,4]triazolo[1,5-a]pyrimidin-7-ol

Compounds of formula I in free or pharmaceutically acceptable salt form are hereinafter referred to alternatively as compounds of the invention.

A compound of the present invention may exist in the form of isomers and mixtures thereof; e.g. optical isomers, diastereoisomers, cis/trans isomers. A compound of the present invention may e.g. contain asymmetric carbon atoms and may thus exist in the form of enatiomers or diastereoisomers and mixtures thereof, e.g. racemates. Substituents at any asymmetric carbon atom may be present in the (R)-, (S)- or (R,S)-configuration, preferably in the (R)- or (S)-configuration. E.g. cis/trans isomers may be present, in case that an aliphatic double bond is present in a compound of the present invention. Isomeric mixtures may be separated as appropriate, e.g. according, e.g. analogously, to a method as conventional, to obtain pure isomers. The present invention includes a compound of the present invention in any isomeric form and in any isomeric mixture.
The present invention also includes tautomers of a compound of the present invention, e.g. a compound of the present invention may be present in the following forms: 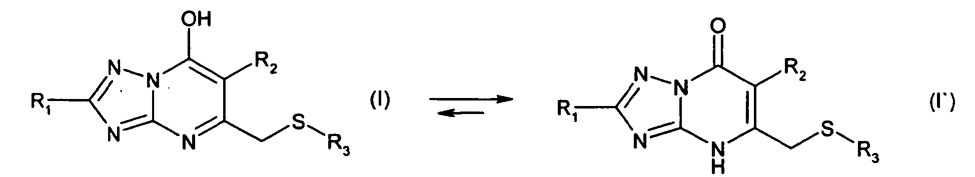 Any compound described herein, e.g. a compound of the present invention, may be prepared as appropriate, e.g. according, e.g. analogously, to a method as conventional, e.g. or as specified herein. Starting materials are known or may be prepared according, e.g. analogously, to a method as conventional or as described herein.

In another aspect the present invention provides a process for the preparation of a compound of the present invention comprising
reacting a compound of formula 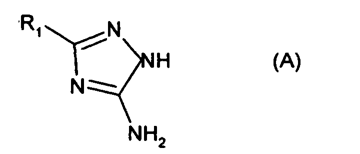 wherein R₁ is as defined above, with a compound of formula 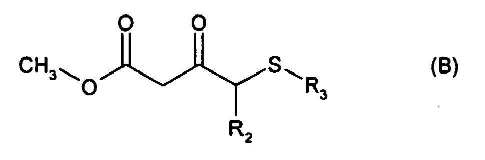 wherein R₂ and R₃ are as defined above, under appropriate conditions, e.g. in the presence of acetic acid at 100°C for 16 hours, to obtain a compound of formula (I) of the present invention.

A compound of formula I thus obtained may be converted into another compound of formula I, e.g. or a compound of formula I obtained in free form may be converted into a salt of a compound of formula I and vice versa.

Compounds of the invention are useful as pharmaceuticals.
Accordingly, the invention also provides a compound of formula (I) in free or pharmaceutically acceptable salt form wherein the substituents are as defined above for use as a pharmaceutical.
In another aspect the present invention provides the use of a compound of formula (I) wherein the substituents are as defined above as a pharmaceutical.
In another aspect the present invention provides the use of a compound of formula (I) wherein the substituents are as defined above in the preparation of a medicament.

The compounds of the invention act as CXCR2 receptor antagonists, thereby inhibiting the infiltration and activation of inflammatory cells, in particular neutrophils, monocytes and CD8+ T cells and mediators involved in chronic obstructive pulmonary disease (COPD). The compounds of the invention therefore provide symptomatic relief and reduce disease progression.
The airways of subject with COPD exhibit an inflammatory response which is predominantly neutrophilic. When the airways are exposed to cigarette smoke macrophages, CD8+ T cells and epithelial cells are activated and release pro-inflammatory mediators, oxidants, cytokines and neutophilic chemotactic factors, IL-8, GROα, ENA-78 and leukotrienes. IL-8, GROα and ENA-78 are selective chemoattractants for neutrophils. In human neutrophils IL-8 binds two distinct receptors with similar affinity, CXCR1 and CXCR2. Closely related chemokines including GROα, β, γ, NAP-2 and ENA-78 bind only to CXCR2. Inhibiting neutrophil recruitment is therefore a recognised therapeutic strategy for treating several lung diseases. Blocking the binding of IL-8, GROα and ENA-78 to the chemokine receptor CXCR2 can provide beneficial effects in patients with COPD by suppressing the infiltration and activation of key inflammatory cells, thereby reducing subsequent tissue damage, mucus secretion, airflow obstruction and disease progression.
The IL-8 and GROα chemokine inhibitory properties of compounds of the invention can be demonstrated in the following ASSAYS:

### Receptor Binding Assay

[¹²⁵I] IL-8 (human recombinant) are obtained from Amersham Pharmacia Biotech, with specific activity 2000 Ci/mmol. All other chemicals are of analytical grade. Human recombinant CXCR2 receptor expressed in Chinese hamster ovary cells (CHO-K1) is purchased from Euroscreen. The Chinese hamster ovary membranes are prepared according to protocol supplied by Euroscreen. Membrane protein concentration is determined using a Bio-Rad protein assay. Assays are performed in a 96-well micro plate format according the method described in White, et al., J Biol Chem., 1998, 273, 10095). Each reaction mixture contains 0.05 mg/ml CXCR2 membrane protein in 20 mM Bis-Tris-propane, pH 8.0, containing 1.2 mM MgSO₄, 0.1 mM EDTA, 25 mM NaCl and 0.03% CHAPS. In addition, compound of interest pre-dissolved in dimethylsulphoxide (DMSO) so as to reach a final concentration of between 10 µM and 0.0005 µM (final concentration of DMSO 2 % (v/v)) is added. Binding is initiated by addition of 0.02 nM ¹²⁵I-IL-8. After 2 hours at room temperature the plate is harvested using a Brandell™ 96-well harvester onto glass fibre filter plate (GF/c) blocked with 1% polyethyleneimine + 0.5% BSA and washed 3 times with 25 mM NaCl, 10 mM TrisHCl, 1 mM MgSO₄, 0.5 mM EDTA, 0.03% CHAPS, pH 7.4. The filter is dried at 50°C overnight. Backseal is applied to the plate and 50 µl of liquid scintillation fluid added. The counts are measured on the Packard Topcount™ scintillation counter.
[³⁵S]-GTPγS binding assay for human CXCR2 receptor using SPA technology
[³⁵S]-GTPγS (with specific activity 1082 Ci/mmol) and wheat germ agglutinin poly vinyl toluene scintillation proximity beads are purchased from Amersham Pharmacia Biotech. The Chinese hamster ovary cell (CHO-K1) membranes expressing human CXCR2 receptors are purchased from Biosignal Packard Inc. All other chemicals are of analytical grade. White non-binding surface 96 well Optiplate™ microplates are obtained from Packard. Recombinant human IL-8 is synthesised, cloned and expressed in *Escherichia coli* as described previously (Lindley I, et al., Proc. Natl. Acad. Sci., 1988, 85(23):9199).
The assay is performed in duplicate in 96 well Optiplate™ microplate in a final volume of 250 µl per well. Compounds are diluted in DMSO (0.5% final concentration) and incubated in 20 mM HEPES buffer pH 7.4 containing 10 mM MgCl₂, 100 mM NaCl, 1 mM EDTA plus 100 nM IL-8, 50 µM GDP and 500 pM [³⁵S]GTPγS per well. SPA beads (1 mg/well final concentration) were pre-mixed with the membranes (10 µg/well final concentration) in assay buffer: 20 mM HEPES buffer pH 7.4 containing 10 mM MgCl₂, 100 mM NaCl, 1 mM EDTA. The bead membrane mixture is added to each well, plates are sealed and incubated at room temperature for 60 minutes. The plate is centrifuged and read on Packard TopCount™ scintillation counter, program [³⁵S dpm] for 1 min/well. Data are expressed as the % response to 100 nM IL-8 minus basal.

### Chemotaxis Assay

The *in vitro* inhibitory properties of these compounds are determined in the neutrophil chemotaxis assay. Assays are performed in a 96-well plate format according to previously published method (Frevert C W, et al., J Immunolog. Methods, 1998, 213, 41). 96-well chemotaxis chambers 5 µm are obtained from Neuro Probe, all cell buffers are obtained from Invitrogen Paisley, UK, dextran -T500 and Ficoll-Paque Plus™ density gradient centrifugation media are purchased from Pharmacia Biotech Buckinghamshire, UK. Calcein-AM dye is obtained from Molecular Probes. Neutrophils are isolated as previously described (Haslett, C., et al. Am J Path., 1985, 119:101). Citrated whole blood is mixed with 4% (w/v) dextran-T500 and allowed to stand on ice for 30 minutes to remove erythrocytes. Granulocytes (PMN) are separated from peripheral blood mononuclear cells by layering 15 ml of cell suspension onto 15 ml Ficoll-Paque PLUS density gradient and centrifuged at 250 xg for 25 minutes. Following centrifugation any erythrocytes contamination of PMN pellet is removed by hypotonic shock lysis using 10 ml ice-cold endotoxin-free sterile water for 50 seconds and neutralised with 10 ml of cold 2x phosphate buffered saline. Isolated neutrophils (1 x10⁷) are labelled with the fluorochrome calcein-AM (5 µg) in a total volume of 1 ml and incubated for 30 minutes at 37°C. The labelled cells are washed with RPMI without phenol red + 0.1% bovine serum albumin, prior to use the cells are counted and adjusted to a final concentration of 5 x 10⁶ cells /ml. The labelled neutrophils are then mixed with test compounds (0.001-1000 nM) diluted in DMSO (0.1 % final concentration) and incubated for 10 minutes at room temperature. The chemoattractants (29 µl) are placed in the bottom chamber of a 96-well chemotaxis chamber at a concentration between (0.1-5 nM). The polycarbonate filter (5 µm) is overlaid on the plate, and the cells (25 µl) are loaded on the top filter. The cells are allowed to migrate for 90 minutes at 37° C in a humidified incubator with 5% CO₂. At the end of the incubation period, migrated cells are quantified using a multi-well fluorescent plate reader (Fluroskan II™, Labsystems) at 485 nm excitation and 538 nm emission. Each compound is tested in quadruplet using 4 different donors. Positive control cells, i.e. cells that have not been treated with compound, are added to the bottom well. These represent the maximum chemotactic response of the cells. Negative control cells, i.e. those that have not been stimulated by a chemoattractant, are added to the bottom chamber. The difference between the positive control and negative control represents the chemotactic activity of the cells.

The compounds of the Examples herein below generally have IC₅₀ values below 10 µM in the [³⁵S]-GTPγS binding assay. For instance, the compounds of Examples 1 and 4 have IC₅₀ values of 0.3 and 2.4 µM, respectively.

Having regard to their inhibition of binding of CXCR2, compounds of the invention are useful in the treatment of conditions or diseases mediated by CXCR2, for example inflammatory or allergic conditions or diseases, particularly chronic obstructive pulmonary airways or lung disease (COPD, COAD or COLD), including chronic bronchitis or dyspnea associated therewith, emphysema, bronchiolitis obliterans syndrome and severe asthma.
Compounds of the present invention are further useful in the treatment of various diseases, such as cancer, e.g. ovarian cancer, prostate cancer, melanoma including metastatic melanoma, lung cancer, e.g. non small cell lung cancer, renal cell carcinoma; tumour angiogenesis, ischaemia/reperfusion injury, delayed graft function, osteoarthritis, myeloid metaplasia with myelofibrosis, Adenomyosis, contact hypersensitivity (skin) and in wound healing.
Treatment in accordance with the invention may be symptomatic or prophylactic.

Prophylactic efficacy in the treatment of chronic bronchitis or COPD will be evidenced by reduced frequency or severity, will provide symptomatic relief and reduce disease progression, improvement in lung function. It may further be evidenced by reduced requirement for other, symptomatic therapy, i.e. therapy for or intended to restrict or abort symptomatic attack when it occurs, for example anti-inflammatory (e.g. corticosteroid) or bronchodilatory.

Other inflammatory or obstructive airways diseases and conditions to which the invention is applicable include acute lung injury (ALI), acute/adult respiratory distress syndrome (ARDS), idiopathic pulmonary fibrosis, fibroid lung, airway hyperresponsiveness, dyspnea, pulmonary fibrosis, allergic airway inflammation, small airway disease, lung carcinoma, acute chest syndrome in patients with sickle cell disease and pulmonary hypertension, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Compounds of the invention are also useful for treating respiratory viral infections, which exacerbate underlying chronic conditions such as asthma, chronic bronchitis, COPD, otitis media, and sinusitis. The respiratory viral infection treated may be associated with secondary bacterial infection, such as otitis media, sinusitis or pneumonia.

Compounds of the invention are also useful in the treatment of inflammatory conditions of the skin, for example psoriasis, atopic dermatitis, lupus erythematosus, and other inflammatory or allergic conditions of the skin.

Compounds of the invention may also be used for the treatment of other diseases or conditions, in particular diseases or conditions having an inflammatory component, for example, diseases affecting the nose including allergic rhinitis, e.g. atrophic, chronic, or seasonal rhinitis, inflammatory conditions of the gastrointestinal tract, for example inflammatory bowel disease such as ulcerative colitis and Crohn's disease, diseases of the bone and joints including rheumatoid arthritis, psoriatic arthritis, and other diseases such as atherosclerosis, multiple sclerosis, and acute and chronic allograft rejection, e.g. following transplantation of heart, kidney, liver, lung or bone marrow.

Compounds of the invention are also useful in the treatment of endotoxic shock, glomerulonephritis, cerebral and cardiac ischemia, Alzheimer's disease, cystic fibrosis, virus infections and the exacerbations associated with them, acquired immune deficiency syndrome (AIDS), multiple sclerosis (MS), *Helicobacter pylori* associated gastritis, and cancers, particularly the growth of ovarian cancer.

Compounds of the invention are also useful for treating symptoms caused by viral infection in a human which is caused by the human rhinovirus, other enterovirus, coronavirus, herpes viruses, influenza virus, parainfluenza virus, respiratory syncytial virus or an adenovirus.

Compounds of the invention are also useful for treating diseases such as pancreatitis, Behcet's disease and hepatobiliary diseases associated with reactive bile ductule, such as chronic viral hepatitis, liver cirrhosis, sepsis, extrahepatic biliary obstruction, fulminant hepatitis, primary biliary cirrhosis and primary sclerosing cholangitis.

The effectiveness of a compound of the invention in inhibiting inflammatory conditions, for example in inflammatory airways diseases, may be demonstrated in an animal model, e.g. mouse, rat or rabbit model, of airway inflammation or other inflammatory conditions, for example as described by Wada et al, J. Exp. Med (1994) 180:1135-40; Sekido et al, Nature (1993) 365:654-57; Modelska et al., Am. J. Respir. Crit. Care. Med (1999) 160:1450-56; and Laffon et al (1999) Am. J. Respir. Crit. Care Med. 160:1443-49.

The compounds of the invention are also useful as co-therapeutic compounds for use in combination with other drug substances such as anti-inflammatory, bronchodilatory, antihistamine or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. A compound of the invention may be mixed with the other drug substance in a fixed pharmaceutical composition or it may be administered separately, before, simultaneously with or after the other drug substance. Accordingly the invention includes a combination of a compound of the invention as hereinbefore described with an anti-inflammatory, bronchodilatory, antihistamine or anti-tussive drug substance, said compound of the invention and said drug substance being in the same or different pharmaceutical composition.

Suitable anti-inflammatory drugs include steroids, in particular glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920; non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935 and WO 04/26248; LTD4 antagonists such as montelukast and zafirlukast; PDE4 inhibitors such cilomilast (Ariflo® GlaxoSmithKline), Roflumilast (Byk Gulden), V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SeICID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839, WO 04/005258, WO 04/018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607 and WO 04/037805; A_{2A} agonists such as those described in EP 1052264, EP 1241176, EP 409595A2, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, and WO 03/086408; and A_{2B} antagonists such as those described in WO 02/42298.

Suitable bronchodilatory drugs include anticholinergic or antimuscarinic compounds, in particular ipratropium bromide, oxitropium bromide, tiotropium salts and CHF 4226 (Chiesi), and glycopyrrolate, but also those described in EP 424021, US 3714357, US 5171744, WO 01/04118, WO 02/00652, WO 02/51841, WO 02/53564, WO 03/00840, WO 03/33495, WO 03/53966, WO 03/87094, WO 04/018422 and WO 04/05285; and beta-2 adrenoceptor agonists such as albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, preferably compounds of the Examples thereof, especially a compound of formula 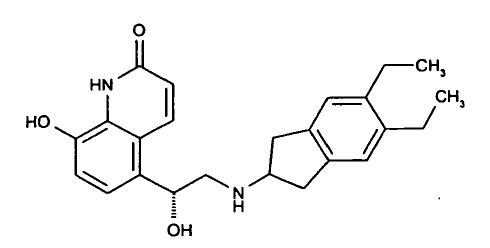 and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 and WO 04/80964.
Such antihistamine drug substances include cetirizine hydrochloride, acetaminophen, clemastine fumarate, promethazine, loratidine, desloratidine, diphenhydramine and fexofenadine hydrochloride.

Combinations of compounds of the invention and anticholinergic or antimuscarinic compounds, steroids, beta-2 agonists, PDE4 inhibitors, dopamine receptor agonists, LTD4 antagonists or LTB4 antagonists may also be used. Other useful combinations of compounds of the invention with anti-inflammatory drugs are those with other antagonists of chemokine receptors, e.g. CCR-1, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzocyclohepten-8-yl]carbonyl]amino]phenyl]-methyl]-tetrahydro-N,N-dimethyl-2H-pyran-4-aminium chloride (TAK-770), CCR-5 antagonists described in US 6166037 (particularly claims 18 and 19), WO 0066558 (particularly claim 8), and WO 0066559 (particularly claim 9).

In accordance with the foregoing, the invention also provides a method for the treatment of a condition or disease mediated by CXCR2, for example an inflammatory or allergic condition, particularly an inflammatory or obstructive airways disease, which comprises administering to a subject, particularly a human subject, in need thereof an effective amount of a compound of formula I in a free or pharmaceutically acceptable salt form as hereinbefore described. In another aspect the invention provides the use of a compound of formula I, in free or pharmaceutically acceptable salt form, as hereinbefore described for the manufacture of a medicament for the treatment of a condition or disease mediated by CXCR2, for example an inflammatory or allergic condition or disease, particularly an inflammatory or obstructive airways disease.

The compounds of the invention may be administered by any appropriate route, e.g. orally, for example in the form of a tablet or capsule; parenterally, for example intravenously; by inhalation, for example in the treatment of inflammatory or obstructive airways disease; intranasally, for example in the treatment of allergic rhinitis; topically to the skin, for example in the treatment of atopic dermatitis; or rectally, for example in the treatment of inflammatory bowel disease.

In a further aspect, the invention also provides a pharmaceutical composition comprising as active ingredient a compound of formula (I) in free or pharmaceutically acceptable salt form, optionally together with a pharmaceutically acceptable diluent or carrier therefor. The composition may contain a co-therapeutic compound such as an anti-inflammatory bronchodilatory or antihistamine drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula I having a particle diameter up to 10 microns, optionally together with a diluent or carrier, such as lactose, of the desired particle size distribution and a compound that helps to protect against product performance deterioration due to moisture, e.g. magnesium stearate. When the composition comprises a nebulised formulation, it preferably contains, for example, the compound of formula I either dissolved, or suspended, in a vehicle containing water, a co-solvent such as ethanol or propylene glycol and a stabiliser, which may be a surfactant.

The invention includes (A) a compound of the invention in inhalable form, e.g. in an aerosol or other atomisable composition or in inhalable particulate, e.g. micronised form, (B) an inhalable medicament comprising a compound of the invention in inhalable form; (C) a pharmaceutical product comprising such a compound of the invention in inhalable form in association with an inhalation device; and (D) an inhalation device containing a compound of the invention in inhalable form.

Dosages of compounds of the invention employed in practising the present invention will of course vary depending, for example, on the particular condition to be treated, the effect desired and the mode of administration. In general, suitable daily dosages for administration by inhalation are of the order of 0.01 to 1 mg/kg per day while for oral administration suitable daily doses are of the order of 0.005 to 100 mg/kg of total body weight. The daily parenteral dosage regimen about 0.001 to about 80 mg/kg of total body weight. The daily topical dosage regimen will preferably be from 0.1 mg to 150 mg, administered one to four, preferably two or three times daily.

In another aspect the present invention provides a compound of formula 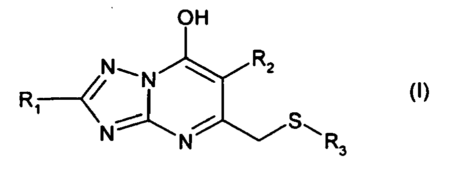 wherein
R₁ and R₂ independently are hydrogen, (C₁₋₈)alkyl, (C₃₋₈)cycloalkyl, (C₁₋₈)alkylthio, (C₆₋₁₈)aryl, (C₁₋₈)alkyl(C₆₋₁₈)aryl,
R₃ is unsubstituted (C₆₋₁₈)aryl, unsubstituted heterocyclyl having 5 to 6 ring members and 1 to 4 heteroatoms selected from N, O, S, or
(C₆₋₁₈)aryl or heterocyclyl having 5 to 6 ring members and 1 to 4 heteroatoms selected from N, O, S one or morefold substituted by (C₁₋₆)alkyl, halogen, halo(C₁₋₆)alkyl, halo(C₁₋₆)alkoxy, cyano, phenyl, heterocyclyl having 5 to 6 ring members and 1 to 4 heteroatoms selected from N, O, S,
or a pharmaceutically acceptable salt or solvate thereof.

In the following examples all temperatures are in degree (°) Celsius.

General Conditions for characterization data of exemplified compounds:
Mass spectra are run on an open access Waters 600/ZQ HPLC/Mass Spectrometer system using electrospray ionization. [M+H]⁺ refers to mono-isotopic molecular weights.

The following ABBREVIATIONS are used in the examples:
- AcOH: acetic acid
- DABCO: 1,4-diazabicyclo[2.2.2]octane
- DBU: 1,8-diazabicyclo[5.4.0]undec-7-ene
- DCM: dichloromethane
- DMF: N,N-dimethylformamide
- EtOAc: ethylacetate
- EtOH: ethanol
- Et₂O: diethyl ether
- MeCN: acetonitrile
- MeOH: methanol
- NaOMe: sodium methoxide
- THF: tetrahydrofuran.
- RT: room temperature

### EXAMPLES:

### Example 1:

### 2-Benzyl-5-(2,3-difluorophenylsulfanylmethyl)-[1,2,4]triazolo[1,5-a]pyrimidin-7-ol

0.675 g of 4-(2,3-Difluorophenylsulfanyl)-3-oxo-butyric acid methyl ester and 0.451 g of 5-benzyl-2H-[1,2,4]triazol-3-ylamine are dissolved in 10 nl of AcOH and stirred at 100° for 5 hours. On cooling, the mixture obtained is diluted with 20 ml of EtOAc, washed 2x with 20 ml of H₂O/brine and dried. The residue obtained is filtered and solvent is evaporated. The product obtained is stirred with Et₂O at RT for 16 hours. A precipitate obtained is filtered off and dried. 2-Benzyl-5-(2,3-difluorophenylsulfanylmethyl)-[1,2,4]triazolo[1,5-a]pyrimidin-7-ol is obtained. **[M+H]⁺**: 385/386

Examples 2 to 17 are prepared in an analagous way to Example 1 but using the appropriate starting materials.

| **EX.** | **Structure** | **[M+H]⁺ (unless given otherwise)** |
|---|---|---|
| **2** | 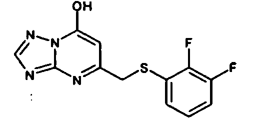 | 294/295 |
| **3** | 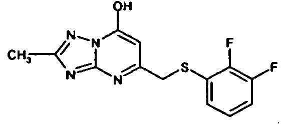 | 309/310 |
| **4** | 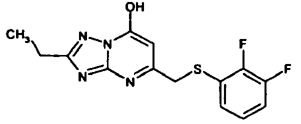 | 323/324 |
| **5** | 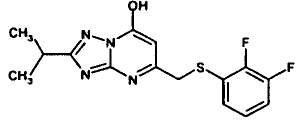 | 337/338 |
| **6** | 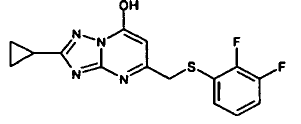 | 335/336 |
| **7** | 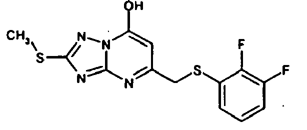 | 341/342 |
| **8** | 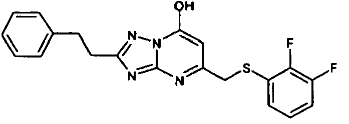 | 399 |
| **9** | 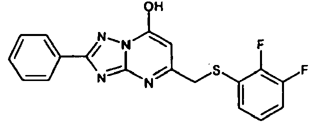 | 371 |
| **10** | 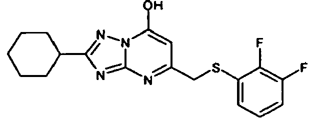 | 377 |
| **11** | 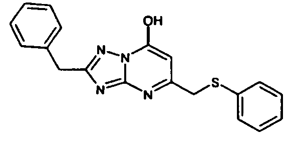 | 349 |
| **12** | 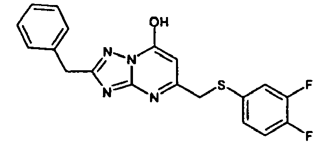 | 385 |
| **13** | 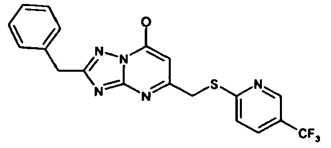 | 418 |
| **14** | 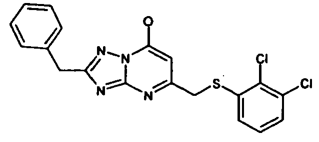 | 417/420 |
| **15** | 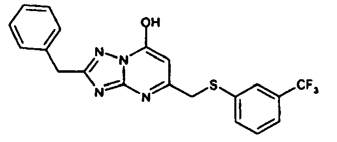 | 417/418 |
| **16** | 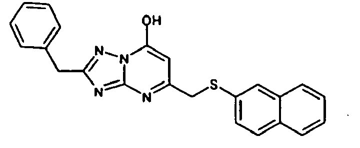 | 399 |
| **17** | 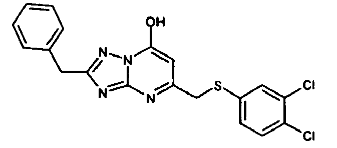 | 417 |

### Example 18:

### 2-Benzyl-5-(3,4-dimethyl-phenylsulfanylmethyl]-[1,2,4]triazolo[1,5-a]pyrimidin-7-ol

A solution of 50 mg of 2-benzyl-5-chloromethyl-[1,2,4]triazolo[1,5-a]pyrimidin-7-ol (Intermediate C) in 1 ml of MeCN is treated with 28 mg of DBU and 25 mg of 3,4-dimethylbenzenethiol. The reaction mixture obtained is stirred at RT for 16 hours and diluted with 1 ml of 1 M HCl and 2 ml of DCM. The organic portion obtained is isolated using a phase separation cartridge, washed with H₂O and solvent is evaporated. The crude residue obtained is triturated with Et₂O, the precipitate obtained is collected by filtration and the filtrate obtained is dried. 2-Benzyl-5-(3,4-dimethyl-phenylsulfanylmethyl)-[1,2,4]triazolo[1,5-a]pyrimidin-7-ol is obtained.

Examples 19 to 27 are prepared in an analagous way to Example 18, but using the appropriate starting materials.

| **EX.** | **Structure** | **[M+H]⁺ (unless given otherwise)** |
|---|---|---|
| **19** | 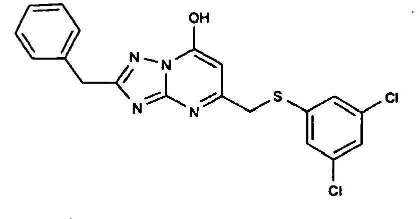 | 417/421 |
| **20** | 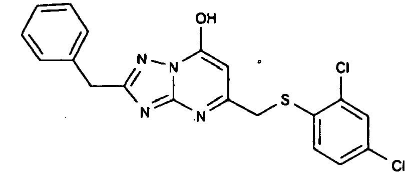 | 417/421 |
| **21** | 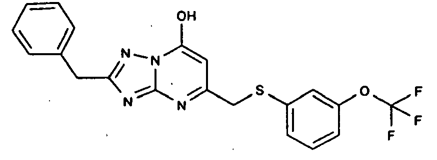 | 433 |
| **22** | 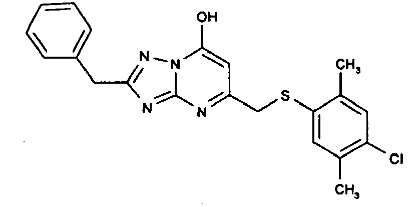 | 411/413 |
| **23** | 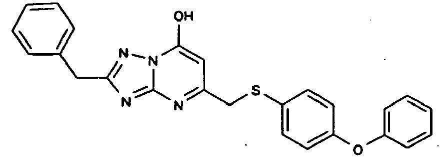 | 441 |
| **24** | 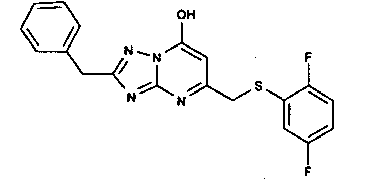 | 385 |
| **25** | 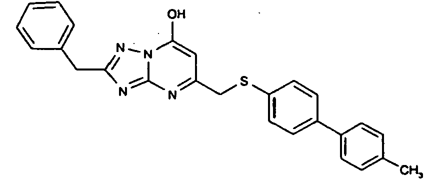 | 439 |
| **26** | 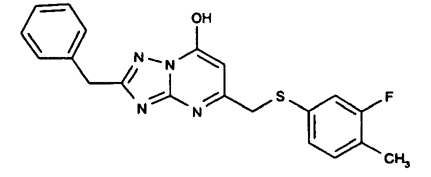 | 381 |
| **27** | 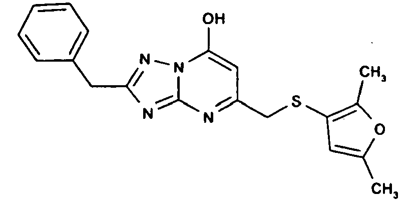 | 367 |

### Preparation of Intermediates

### Intermediate A: 4-(2,3-Difluoro-phenylsulfanyl)-3-oxo-butyric acid methyl ester

308 mg of solid Na are added portionwise to anhydrous 20 ml of MeOH under an inert atmosphere of argon at RT. After all the Na is dissolved the reaction mixture obtained is cooled to 0°. 2.22 g of 4-chloroacetoacetate and 1.96 g of 2,3-difluoro-benzenethiol (Intermediate B) are added. The reaction mixture obtained is warmed to RT and stirred overnight. Solvent is evaporated and the evaporation residue obtained is dissolved in EtOAc, washed with saturated NH₄Cl solution, dried, filtered and concentrated. Purification by column chromatography on silica with EtOAc:iso-hexane (10-30%) may be carried out. 4-(2,3-Difluorophenylsulfanyl)-3-oxo-butyric acid methyl ester is obtained.

### Intermediate B: 2,3-Difluoro-benzenethiol

### a) Dimethyl-thiocarbamic acid O-(2,3-difluoro-phenyl) ester

A solution of 8.39 g of 2,3-difluorophenol in 100 ml of anhydrous DMF is treated with 14.45 g of DABCO and 12 g of dimethylthiocarbomyl chloride under an inert atmosphere of argon. The reaction mixture obtained is heated at 35° for 30 minutes and at 75° for 4 hours. The reaction mixture obtained is cooled to RT, diluted with 200 ml of H₂O and stirred at RT for 48 hours. The precipitate obtained is collected by filtration, the filtrate obtained is washed with H₂O and solvent is evaporated. Dimethyl-thiocarbamic acid O-(2,3-difluoro-phenyl) ester is obtained.

### b) Dimethyl-thiocarbamic acid S-(2,3-difluoro-phenyl) ester

5.55 g of Dimethyl-thiocarbamic acid O-(2,3-difluoro-phenyl) ester, 20 ml of dowtherm^{®} A (eutectic mixture of 25.6% diphenyl + 73.5% of diphenyl oxide) and 70 mg of K₂CO₃ are mixed together and heated at 250° for 3.5 hours. After cooling to RT the reaction mixture obtained is purified by flash chromatography on silica eluting initially with iso-hexanes to remove the dowtherm^{®} then increasing the gradient to iso-hexanes:EtOAc (3:1). The appropriate fractions are combined, concentrated and dried. The title compound is obtained.

### c) 2,3-Difluoro-benzenethiol

620 mg of solid Na are added portion wise to 90 ml of anhydrous MeOH under an inert atmosphere of argon to give a solution of NaOMe. The solution obtained is treated with 3.8 g of dimethyl-thiocarbamic acid S-(2,3-difluoro-phenyl) ester in 9 ml of MeOH and the reaction mixture obtained is heated at reflux for 3 hours. After cooling to RT the reaction mixture obtained is stirred overnight for 16 hours, solvent is evaporated and the residue obtained is diluted with Et₂O and washed 2x with 2M HCl. The organic residue obtained is dried, filtered and the residue obtained is concentrated. The title compound is obtained.
Accordingly, a range of benzenethiols may be prepared using the appropriate starting materials.

### Intermediate C: 2-Benzyl-5-chloromethyl-[1,2,4]triazolo[1,5-a]pyrimidin-7-ol

A solution of 2 g of 4-chloroacetoacetate and 3.45 g of 5-benzyl-1H-1,2,4-triazole-3-amine 60 ml of in AcOH is heated at 80° for 20 hours. On cooling to RT the reaction mixture obtained is diluted with EtOAc and H₂O, the organic portion is separated, dried, filtered and concentrated. The residue obtained is triturated with Et₂O, filtered and dried.
The title compound is obtained. [M+H]⁺ 274.

### Intermediate D: 5-Morpholin-4-yl-4H-[1,2,4]triazol-3-ylamine

### a) Methyl N-cyanomorpholine-4-carbimidothioate

A solution of 500 mg of N-cyanodithiocarbonimidate in 4 ml of DCM is treated with 326.7 mg of morpholine and the reaction mixture obtained is stirred at RT for 30 minutes. The reaction mixture obtained is diluted with DCM and washed with H₂O, the organic portion obtained is dried, filtered and concentrated. The title compound is obtained.

### b) 5-Morpholin-4-yl-4H-[1,2,4]triazol-3-ylamine

A solution of 490 mg of methyl N-cyanomorpholine-4-carbimidothioate and 188 mg of hydrazine monohydrate in 4 ml of MeCN is heated using microwave radiation at 150° for 10 minutes and the reaction mixture obtained is concentrated.
The title compound is obtained. [M+H]⁺170

### Intermediate E: N*5*,N*5*-Dimethyl-1H-[1,2,4]triazole-3,5-diamine

### a) Methyl N'-cyano-N,N-dimethylcarbamimidothioate

A solution of 500 mg of N-cyanodithiocarbonimidate in 8 ml of DCM is treated with 216 mg of 2M dimethylamine in THF as a solution in 4 ml of DCM and the reaction mixtureobtained is heated at 50° for 1 hour. After cooling to RT the reaction mixture obtained is diluted with DCM and washed with 1M HCl, the aqueous phase is neutralized and extracted with DCM. The combined organic portions obtained are dried, filtered and concentrated. Purification of the crude residue by flash chromatography on silica eluting with EtOAc:*iso*-hexanes (20% to 50% EtOAc) may be carried out. The title compound is obtained.

### b) N*5*,N*5*-Dimethyl-1H-[1,2,4]triazole-3,5-diamine

This compound is prepared analogously to Intermediate D, step b, by using the appropriate starting materials.

### Intermediate F: N-Cyclohexyl-N-methyl-4H-[1,2,4]triazole-3,5-diamine

### a) Methyl N'-cyano-N-cyclohexyl-N-methylcarbamimidothioate

This compound is prepared analogously to Intermediate D, step 1, by using the appropriate starting materials.

### b) N-Cyclohexyl-N-methyl-4H-[1,2,4]triazole-3,5-diamine

A solution of 1.44 g of methyl N'-cyano-N-cyclohexyl-N-methylcarbamimidothioate and 375 mg of hydrazine monohydrate in 40 ml of MeCN is heated at 100° for 5 hours. After cooling to RT the reaction mixture obtained is concentrated *in vacuo* and the crude residue is purified by flash chromatography on silica eluting with MeOH:CHCl₃ (7% to 10% MeOH). The appropriate fractions are combined, solvent is evaporated and the resulting residue is triturated with MeCN:iso-hexanes. The title compound is obtained. [M+H]⁺196.

## Claims

1. A compound of formula 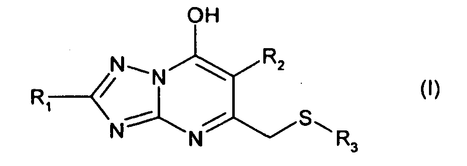 wherein
R₁ and R₂ independently are hydrogen, (C₁₋₈)alkyl, (C₃₋₈)cycloalkyl, (C₁₋₈)alkylthio, (C₆₋₁₈)aryl, (C₁₋₈)alkyl(C₆₋₁₈)aryl, (C₆₋₁₈)aryl(C₁₋₈)alkyl, heterocyclyl having 5 to 6 ring members and 1 to 4 heteroatoms selected from N, O, S,
R₃ is (C₆₋₁₈)aryl or heterocyclyl having 5 to 6 ring members and 1 to 4 heteroatoms selected from N, O, S, unsubstituted or 1- or morefold substituted by (C₁₋₆)alkyl, halo(C₁₋₆)alkyl, halogen, halo(C₁₋₆)alkoxy, cyano, phenyl, heterocyclyl having 5 to 6 ring members and 1 to 4 heteroatoms selected from N, O, S,
or a pharmaceutically acceptable salt or solvate thereof.

2. A compound of any one of claims 1 or 2, wherein
R₁ is hydrogen, methyl, ethyl, i-propyl, cyclopropyl, cyclohexyl, methylthio, phenyl, benzyl, phenethyl,
R₂ is hydrogen,
R₃ is unsubstituted naphthyl or phenyl, phenyl 1- to 3-fold substituted by methyl, chloro, fluoro, trifluoromethyl, trifluoromethoxy, phenyl, phenoxy, 4-methylphenyl, unsubstituted naphthyl, pyridinyl substituted by trifluoromethyl, 2-5-dimethylfuran-3-yl.

3. A compound of formula (I) selected from the group consisting of
- 2-Benzyl-5-(2,3-difluorophenylsulfanylmethyl)-[1,2,4]triazolo[1,5-a]pyrimidin-7-ol
- 2-Methyl-5-(4-chlorophenylsulfanylmethyl)-[1,2,4]triazolo[1,5-a]pyrimidin-7-ol
- 2-Methyl-5-(4-methylphenylsulfanylmethyl)-[1,2,4]triazolo[1,5-a]pyrimidin-7-ol
- 5-(2,3-difluorophenylsulfanylmethyl)-[1,2,4]triazolo[1,5-a]pyrimidin-7-ol
- 2-Methyl-5-(2,3-difluorophenylsulfanylmethyl)-[1,2,4]triazolo[1,5-a]pyrimidin-7-ol
- 2-Ethyl-5-(2,3-difluorophenylsulfanylmethyl)-[1,2,4]triazolo[1,5-a]pyrimidin-7-ol
- 2-Isopropyl-5-(2,3-difluorophenylsulfanylmethyl)-[1,2,4]triazolo[1,5-a]pyrimidin-7-ol
- 2-Cyclopropyl-5-(2,3-difluorophenylsulfanylmethyl)-[1,2,4]triazolo[1,5-a]pyrimidin-7-ol
- 2-Methylthio-5-(2,3-difluorophenylsulfanylmethyl)-[1,2,4]triazolo[1,5-a]pyrimidin-7-ol
- 2-Phenethyl-5-(2,3-difluorophenylsulfanylmethyl)-[1,2,4]triazolo[1,5-a]pyrimidin-7-ol
- 2-Phenyl-5-(2,3-difluorophenylsulfanylmethyl)-[1,2,4]triazolo[1,5-a]pyrimidin-7-ol
- 2-Cyclohexyl-5-(2,3-difluorophenylsulfanylmethyl)-[1,2,4]triazolo[1,5-a]pyrimidin-7-ol
- 2-Benzyl-5-phenylsulfanylmethyl-[1,2,4]triazolo[1,5-a]pyrimidin-7-ol
- 2-Benzyl-5-(3,4-difluorophenylsulfanylmethyl)-[1,2,4]triazolo[1,5-a]pyrimidin-7-ol
- 2-Benzyl-5-(5-trifluoromethyl-pyridin-2-yl-sulfanylmethyl)-[1,2,4]triazolo[1,5-a]pyrimidin-7-ol
- 2-Benzyl-5-(2,3-dichlorophenylsulfanylmethyl)-[1,2,4]triazolo[1,5-a]pyrimidin-7-ol
- 2-Benzyl-5-(3-trifluoromethyl-phenylsulfanylmethyl)-[1,2,4]triazolo[1,5-a]pyrimidin-7-ol
- 2-Benzyl-5-(naphthalen-2-yl-sulfanylmethyl)-[1,2,4]triazolo[1,5-a]pyrimidin-7-ol
- 2-Benzyl-5-(3,4-dichlorophenylsulfanylmethyl)-[1,2,4]triazolo[1,5-a]pyrimidin-7-ol
- 2-Benzyl-5-(3,5-dichloro-phenylsulfanylmethyl)-[1,2,4]triazolo[1,5-a]pyrimidin-7-ol
- 2-Benzyl-5-(2,4-dichloro-phenylsulfanyimethyl)-[1,2,4]triazolo[1,5-a]pyrimidin-7-ol
- 2-Benzyl-5-(3-trifluoromethoxy-phenylsulfanylmethyl)-[1,2,4]triazolo[1,5-a]pyrimidin-7-ol
- 2-Benzyl-5-(4-chloro-2,5-dimethyl-phenylsulfanylmethyl)-[1,2,4]triazolo[1,5-a]pyrimidin-7-ol
- 2-Benzyl-5-(4-phenoxy-phenylsulfanylmethyl)-[1,2,4]triazolo[1,5-a]pyrimidin-7-ol
- 2-Benzyl-5-(2,5-difluoro-phenylsulfanylmethyl)-[1,2,4]triazolo[1,5-a]pyrimidin-7-ol
- 2-Benzyl-5-(4'-methyl-biphenyl-3-ylsulfanylmethyl)-[1,2,4]triazolo[1,5-a]pyrimidin-7-ol
- 2-Benzyl-5-(3-fluoro-4-methyl-phenylsulfanylmethyl)-[1,2,4]triazolo[1,5-a]pyrimidin-7-ol and
- 2-Benzyl-5-(2,5-dimethyl-furan-3-ylsulfanylmethyl)-[1,2,4]triazolo[1,5-a]pyrimidin-7-ol

4. A compound of any one of claims 1 to 3 for use as a pharmaceutical.

5. A compound of formula I as defined in any one of claims 1 to 3 for use in the manufacture of a medicament for the treatment of a CXCR2 receptor mediated condition or disease.

6. A compound of formula I as defined in any one of claims 1 to 3 for the manufacture of a medicament for the treatment of an inflammatory or allergic condition or disease, particularly an inflammatory or obstructive airway disease.

7. A pharmaceutical composition comprising as active ingredient a compound of formula (I) of any one of claims 1 to 3 in free or pharmaceutically acceptable salt form, optionally together with a pharmaceutically acceptable diluent or carrier therefor.

8. A pharmaceutical composition of claim 9 together with at least one further pharmaceutically active compound.

## Patentansprüche

1. Verbindung der Formel 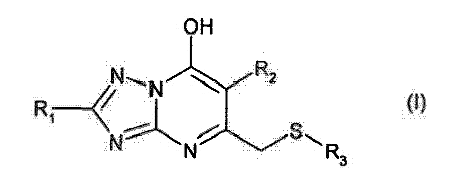 worin
R₁ und R₂ unabgängig voreinander für Wasserstoff, (C₁₋₈)-Alkyl, (C₃₋₈)-Cycloalkyl, (C₁₋₈)-Alkylthio, (C₆₋₁₈)-Aryl, (C₁₋₈)-Alkyl-(C₆₋₁₈)-aryl, (C₆₋₁₈)-Aryl-(C₁₋₈)-alkyl, Heterocyclyl mit 5 bis 6 Ringglieder und 1 bis 4 unter N, O und S ausgewählten Heteroatomen stehen,
R₃ für (C₆₋₁₈)-Aryl oder Heterocyclyl mit 5 bis 6 Ringgliedern und 1 bis 4 unter N, O und S ausgewählten Heteroatomen, das gegebenenfalls 1-oder mehrfach durch (C₁₋₆)-Alkyl, Halogen-(C₁₋₆)-alkyl, HalOgen, Halogen-(C₁₋₆)-alkoxy, Cyano, Phenyl oder Heterocyclyl mit 5 bis 6 Ringgliedern und 1 bis 4 unter N, O und S ausgewählten Heteroatomen substituiert ist, steht,
oder ein pharmazeutisch unbedenkliches Salz oder Solvat davon.

2. Verbindung nach einem der Ansprüche 1 oder 2, wohin
R₁ für Wasserstoff, Methyl, Methyl, Isopropyl, Cyclopropyl, Cyclohexyl, Methylthio, Phenyl, Benzyl oder Phenethyl steht,
R₂ für Wasserstoff steht,
R₃ für unsubstituiertes Naphthyl oder Phenyl, 1-bis 3-fach durch Methyl, Chlor, Fluor, Trifluorethyl, Triflourmethoxy, Phenol, Phenoxy, 4-Methylphenyl oder unsubstituiertes Naphthyl substituierter Phenol, durch Trifluorethyl substituiertes Pyridinyl oder 2,5-Dimethylfuran-3-yl steht.

3. Verbindung der Formel (I), ausgewählt aus der Gruppe bestehend aus:
- 2-Benzyl-5-(2,3-difluorphenylsulfanylmethyl)-[1,2,4]triazolo[1,5-a]pyrimidin-7-ol
- 2-Methyl-5-(4-chlorphenylsulfanylmethyl)-[1,2,4]triazolo[1,5-a]pyrimidin-7-ol
- 2-Methyl-5-(4-methylphenylsulfanylmethyl)-[1,2,4]triazolo[1,5-a]pyrimidin-7-ol
- 5-(2,3-Difluorphenylsulfanylmethyl)-[1,2,4]triazolo[1,5-a]pyrimidin-7-ol
- 2-Methyl-5-(2,3-difluorphenylsulfanylmethyl)-[1,2,4]triazolo[1,5-a]pyrimidin-7-ol
- 2-Ethyl-5 (2,3-difluorphenylsulfanylmethyl)-[1,2,4]triazolo[1,5-a]pyrimidin-7-ol
- 2-Isopropyl-5-(2,3-difluorphenylsulfanylmethyl)-[1,2,4]triazolo[1,5-a]pyrimidin-7-ol
- 2-Cyclopropyl-5-(2,3-difluorphenylsulfanylmethyl)-[1,2,4]triazolo[1,5-a]pyrimidin-7-ol
- 2-Methylthio-5- 2,3-difluorphenylsulfanylmethyl)-[1,2,4]triazolo[1,5-a]pyrimidin-7-ol
- 2-Phenethyl-5-(2,3-difluorphenylsulfanylmethyl)-[1,2,4]triazolo[1,5-a]pyrimidin-7-ol
- 2-Phenyl-5-(2,3-difluorphenylsulfanylmethyl)-[1,2,4]triazolo[1,5-a]pyrimidin-7-ol
- 2-Cyclohexyl-5-(2,3-difluorphenylsulfanylmethyl)-[1,2,4]triazolo[1,5-a]lyrimidin-7-ol
- 2-Benzyl-5-phenylsulfanylmethyl-[1,2,4]triazolo[1,5-a]pyrimidin-7-ol
- 2-Benzyl-5-(3,4-difluorphenylsulfanylmethyl)-[1,2,4]triazolo[1,5-a]pyrimidin-7-ol
- 2-Benzyl-5-(5-trifluormethylpyridin-2-ylsulfanylmethyl)-[1,2,4]triazolo[1,5-a]pyrimidin-7-ol
- 2-Benzyl-5-(2,3-dichlorphenylsulfanylmethyl)-[1,2,4]triazolo[1,5-a]pyrimidin-7-ol
- 2-Benzyl-5-(3-trifluormethylphenylsulfanylmethyl)-[1,2,4]triazolo[1,5-a]pyrimidin-7-ol
- 2-Benzyl-5-(naphthalin-2-ylsulfanylmethyl)-[1,2,4]triazolo[1,5-a]pyrimidin-7-ol
- 2-Benzyl-5-(3,4-dichlorphenylsulfanylmethyl)-[1,2,4]triazolo[1,5-a]pyrimidin-7-ol
- 2-Benzyl-5-(3,5-dichlorphenylsulfanylmethyl)-[1,2,4]triazolo[1,5-a]pyrimidin-7-ol
- 2-Benzyl-5-(2,4-dichlorphenylsulfanylmethyl)-[1,2,4]triazolo[1,5-a]pyrimidin-7-ol
- 2-Benzyl-5-(3-trifluormethoxyphenylsulfanylmethyl)-[1,2,4]triazolo[1,5-a]pyrimidin-7-ol
- 2-Benzyl-5-(4-chlor-2,5-dimethylphenylsulfanylmethyl)-[1,2,4]triazolo[1,5-a]pyrimidin-7-ol
- 2-Benzyl-5-(4-phenoxyphenylsulfanylmethyl)-[1,2,4]triazolo[1,5-a]pyrimidin-7-ol
- 2-Benzyl-5-(2,5-difluorphenylsulfanylmethyl)-[1,2,4]triazolo[1,5-a]pyrimidin-7-ol
- 2-Benzyl-5-(4'-methylbiphenyl-3-ylsulfanylmethyl)-[1,2,4]triazolo[1,5-a]pyrimidin-7-ol
- 2-Benzyl-5-(3-fluor-4-methylphenylsulfanylmethyl)-[1,2,4]triazolo[1,5-a]pyrimidin-7-ol und
- 2-Benzyl-5-(2,5-dimethylfuran-3-ylsulfanylmethyl)-[1,2,4]triazolo[1,5-a]pyrimidin-7-ol

4. Verbindung nach einem der Ansprüche 1 bis 3 zur Verwerdung als Pharmazeutikum.

5. Verbindung der Formel I gemäß einem der Ansprüche 1 bis 3 zur Verwendung bei der Herstellung eines Arzneimittels zur Behandlung eines durch den CXCR2-Rezeptor vermittelten Leidens oder einer durch den CXCR2-Rezeptor vermittelten Erkrankung.

6. Verbindung der Formol I gemäß einem der Ansprüche 1 bis 3 zur Herstellung eines Arzneimittels zur Behandlung eines entzündlichen oder allergischen Leidens oder einer entzündlichen oder allergischen Erkrankung, insbesondere einer entzündlichen oder obstruktiven Atemwegserkrankung.

7. Pharmazeutische Zusammensetzung, die als Wirkstoff eine Verbindung der Formel (I) nach einem der Ansprüche 1 bis 3 in freier Form oder in Form eines pharmazeutisch unbedenkliches Salzes umfasst, gegebenenfalls zusammen mit einem pharmazeutisch unbedenklichen Verdünnungsmittel oder Träger dafür.

8. Pharmazeutische zusammensetzung nach Anspruch 9 zusammen mit mindestens einer weiteren pharmazeutisch wirksamen Verbindung.

## Revendications

1. Composé de formule 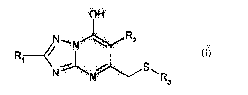 où
R₁ et R₂ représentent indépendamment un atome d'hydrogène ou un groupement alkyle en C₁₋₈, cycloalkyle en C₃₋₈, (alkyle en C₁₋₈)-thio, aryle en C₆₋₁₈, (alkyle en C₁₋₈)-(aryle en C₆₋₁₈), (aryle en C₆₋₁₈)-(alkyle en C₁₋₈), hétérocyclyle comportant 5 à 6 chaînons et 1 à 4 hétéroatomes choisis parmi N, O, S,
R₃ représente un groupement aryle en C₆₋₁₈ ou hétérocyclyle comportant 5 à 6 chaînons et 1 à 4 hétéroatomes choisis parmi N, O, S, non substitué ou substitué une ou plusieurs fois par des groupements alkyle en C₁₋₆, halogénoalkyle en C₁₋₆, halogéno, halogénoalkoxy en C₁₋₆, cyano, phényle, hétérocyclyle comportant 5 à 6 chaînons et 1 à 4 hétéroatomes choisis parmi N, O, S
ou l'un de ses sels ou solvates de qualité pharmaceutique.

2. composé conforme à l'une quelconque des revendications 1 ou 2, où
R₁ représente un atome d'hydrogène ou un groupement méthyle, éthyle, i-propyle, cyclopropyle, cyclohexyle, méthylthio, phényle, benzyle, phénéthyle,
R₂ représente un atome d'hydrogène,
R₃ représente un groupement naphtyle ou phényle non substitué, phényle substitué une à trois fois par des groupements méthyle, chloro, fluoro, trifluorométhyle, trifluorométhoxy, phényle, phénoxy, 4-méthylphényle, naphtyle non substitué, pyridinyle substitué par des groupement trifluorométhyle, 2-5-diméthylfurann-3-yle.

3. Composé de formule (I) choisi dans le groupe constitué par les composés suivants :
- 2-Benzyl-5-(2,3-difluozophénylsulfanylméthyl)-[1,2,4]triazolo[1,5-a]pyrimidin-7-ol
- 2-Méthyl-5-(4-chlorophénylsulfanylmérhyl)-[1,2,4]triazolo[1,5-a]pyrimidin-7-ol
- 2-Méthyl-5-(4-méthylphénylsulfanylméthyl)-[1,2,4]triazolo[1,5-a]pyrimidin-7-ol
- 5-(2,3-difluorophénylsulfanylméthyl)-[1,2,4]triazolo[1,5-a]pyrimidin-7-ol
- 2-Méthyl-5-(2,3-difluorophénylsulfanylméthyl)-[1,2,4]triazolo[1,5-a]pyrimidin-7-ol
- 2-Éthyl-5-(2,3-àifluorophénylsulfanylméthyl)-[1,2,4]triazolo[1,5-a]pyrimidin-7-ol
- 2-Isopropyl-5-(2,3-difluorophénylsulfanylméthyl)-[1,2,4]triazolo[1,5-a]pyrimidin-7-ol
- 2-Cyclopropyl-5-(2,3-difdluorophénylsulfanylméthyl)-[1,2,4]triazolo[1,5-a]pyrimidin-7-ol
- 2-Méthylthio-5-(2,3-difluorophénylsulfanylméthyl)-[1,2,4]triazolo[1,5-a]pyrimidin-7-ol
- 2-Phénéthyl-5-(2,3-difluorophénylsulfanylméthyl)-[1,2,4]triazolo[1,5-a]pyrimidin-7-ol
- 2-Phényl-5-(2,3-difluorophénylsulfanylméthyl)-[1,2,4]triazolo[1,5-a]pyrimidin-7-ol
- 2-Cyclohexyl-(2,3-difluorophénylsulfanylméthyl)-[1,2,4]triazolo[1,5-a]pyrimidin-7-ol
- 2-Benzyl-5-phénylsulfanylméthyl-[1,2,4]triazolo[1,5-a]pyrimidin-7-ol
- 2-Benzyl-5-(3,4-difluorophénylsulfanylméthyl)-[1,2,4]triazolo[1,5-a]pyrimidin-7-ol
- 2-Benzyl-5-(5-trifluorométhyl-pyridin-2-ylsulfanylméthyl)-[1,2,4]triazolo[1,5-a]pyrimidin-7-ol 2-Benzyl-5-(2,3-dichlorophénylsulfanylméthyl)-[1,2,4]triazolo[1,5-a]pyrimidin-7-ol
- 2-Benzyl-5-(3-trifluorométhyl-phénylsulfanylméthyl)-[1,2,4]triazolo[1,5-a]pyrimidin-7-ol
- 2-Benzyl-5-(naphtalén-2-yl-sulfanylméthyl)-[1,2,4]triazolo[1,5-a]pyrimidin-7-ol
- 2-Benzyl-5-(3,3-dichlorophénylsulfanylméthyl)-[1,2,4]triazolo[1,5-a]pyrimidin-7-ol
- 2-Benzyl-5-(3,5-dichlorophénylsulfanylméthyl)-[1,2,4]triazolo[1,5-a]pyrimidin-7-ol
- 2-Benzyl-5-(2,4-dichlorophénylsulfanylméthyl)-[1,2,4]triazolo[1,5-a]pyrimidin-7-ol
- 2-Benzyl-5-(3-trifluorométhoxy-phénylsulfanylméthyl)-[1,2,4]triaaolo[1,5-a]pyrimidin-7-ol
- 2-Benzyl-5-(4-chloro-2,5-diméthyl-phénylsulfanylméthyl)-[1,2,4]triazolo[1,5-a]pyrimdin-7-ol
- 2-Benzyl-5-(4-phénoxy-phénylsulfanylméthyl)-[1,2,4]triazolo[1,5-a]pyrimidin-7-ol
- 2-Benzyl-5-(2,5-difluoro-phénylsulfanylméthyl)-[1,2,4]triazolo[1,5-a]pyrimidin-7-ol
- 2-Benzyl-5-(4'-méthyl-biphényl-3-ylsulfanylméthyl)-[1,2,4]triazolo[1,5-a]pyrimidin-7-ol
- 2-Benzyl-5-(3-fluoro-4-méthyl-phénylsulfanylméthyl)-[1,2,4]triazolo[1,5-a]pyrimidin-7-ol et
- 2-Benzyl-5-(2,5-diméthyl-furann-3-ylsulfanylméthyl)-[1,2,4]triazolo[1,5-a]pyrimidin-7-ol

4. composé conforme à l'une quelconque des revendications 1 à 3 pour emploi en tant que produit pharmaceutique.

5. composé de formule I conforme à l'une quelconque des revendications 1 à 3 pour emploi dans la fabrication d'un médicament destiné au traitement d'un état pathologique ou d'une pathologie faisant intervenir le récepteur CXCR2.

6. Composté de formule I conforme à l'une quelconque des revendications 1 à 3 dans la fabrication d'un médicament destiné au traitements d'un état pathologique ou d'une pathologie inflammatoire ou allergique, particulièrement d'une patrologie inflammatoire ou obstructive des voies respiratoires.

7. composition pharmaceutique comprenant un principe actif qui est un composé de formule (I) selon l'une quelconque des revendications 1 à sous forme libre ou sous forme de sel de qualité pharmaceutique, et éventuellement avec un diluant ou vecteur de qualité pharmaceutique correspondants,

8. Composition pharmaceutique conforme à la revendication 7 avec au moins un principe actif pharmaceutique supplémentaire.
